(19) Europäisches Patentamt
European Patent Office
Office européen des brevets

(11) **EP 4 205 650 A1**

(12) **EUROPEAN PATENT APPLICATION**
published in accordance with Art. 153(4) EPC

(43) Date of publication:
**05.07.2023 Bulletin 2023/27**

(51) International Patent Classification (IPC):
**_A61B 5/1455_** (2006.01)

(21) Application number: **21865730.2**

(86) International application number:
**PCT/CN2021/110415**

(22) Date of filing: **04.08.2021**

(87) International publication number:
**WO 2022/052674 (17.03.2022 Gazette 2022/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.09.2020 CN 202010943525**

(71) Applicant: **Huawei Technologies Co., Ltd.
Shenzhen, Guangdong 518129 (CN)**

(72) Inventors:
• **LI, Yue**
**Shenzhen, Guangdong 518129 (CN)**

• **YE, Jilong**
**Shenzhen, Guangdong 518129 (CN)**
• **LI, Weinan**
**Shenzhen, Guangdong 518129 (CN)**
• **YANG, Bin**
**Shenzhen, Guangdong 518129 (CN)**
• **CHEN, Wenjuan**
**Shenzhen, Guangdong 518129 (CN)**

(74) Representative: **Huawei European IPR
Huawei Technologies Duesseldorf GmbH
Riesstraße 25
80992 München (DE)**

(54) **BLOOD OXYGEN TESTING METHOD AND APPARATUS WITH MULTIPLE PHOTOELECTRIC DETECTORS CONNECTED IN PARALLEL**

(57) This application provides a blood oxygen detection method and apparatus with multiple photoelectric detectors connected in parallel, relates to the field of blood oxygen detection technologies, and can reduce a drive current of a PPG detection module when an electronic device detects blood oxygen, thereby reducing power consumption of blood oxygen detection and improving user experience. The method includes: An electronic device determines, based on a plurality of pieces of detection data, a category of a scenario in which the electronic device currently performs blood oxygen detection, where the detection data is used for describing whether detection currents output by corresponding photoelectric detectors are abnormal, or the detection data is used for describing a current wearing state of the electronic device; and determines, based on the scenario category, to perform calculation based on a detection current output by at least one of the multiple PDs to obtain oxygen saturation, and/or prompts, based on the detection data, a user to re-adjust a wearing state.

An electronic device determines, based on a plurality of pieces of detection data, a category of a scenario in which blood oxygen detection is currently performed — S201

The electronic device determines, from multiple PDs based on the scenario category, a detection current output by at least one PD, to obtain oxygen saturation through calculation, and/or prompts, based on the detection data, a user to re-adjust a wearing state — S202

FIG. 2

## Description

This application claims priority to Chinese Patent Application No. 202010943525.4, filed with the China National Intellectual Property Administration on September 9, 2020 and entitled "BLOOD OXYGEN DETECTION METHOD AND APPARATUS WITH MULTIPLE PHOTOELECTRIC DETECTORS CONNECTED IN PARALLEL", which is incorporated herein by reference in its entirety.

## TECHNICAL FIELD

This application relates to the field of blood oxygen detection technologies, and in particular to a blood oxygen detection method and apparatus with multiple photoelectric detectors connected in parallel.

## BACKGROUND

Currently, more types of electronic devices enter work and life of users. For example, a wearable device may be used to monitor a health index of a user. For example, a user may measure oxygen saturation and view a measurement result by wearing a smartwatch. The oxygen saturation (Oxygen Saturation, SpO2) is a percentage of a capacity of oxyhemoglobin (HbO2) in the blood in a total hemoglobin (hemoglobin, Hb) capacity, that is, a concentration of blood oxygen in the blood.

A technical principle based on which the electronic device performs blood oxygen detection includes a photoplethysmography (Photo Plethysmo Graphy, PPG) method, which is simply a non-invasive detection method for detecting a blood volume change in human tissue through photoelectric conversion. Light beams of a specific wavelength are irradiated to the skin surface, and the light beams are transmitted to a photoelectric detector (Photoelectric Detector, PD) through human tissue such as skin, muscles, bones, and blood of the detected body in a transmission or reflection manner. In this process, an optical signal is absorbed and attenuated, intensity of the light received by the detector is weakened, and the light intensity change signal may be converted into an electrical PPG signal. The absorption of light by tissue such as skin, muscles, veins, and bones is basically constant in the entire blood circulation, but the absorption of light by arteries changes with the pulse period, and therefore, the intensity of light received by an optical receiver fluctuates accordingly. The PPG signal includes an alternating current (Alternating Current, AC) signal and a direct current (Direct Current, DC) signal. However, the absorption coefficients of oxyhemoglobin HbO2 and reduced hemoglobin Hb that is not oxygenated in human blood are different for different wavelengths of light. Therefore, bispectrum quantitative analysis may be performed by emitting more than two types of light beams, that is, a ratio of a DC component to an AC component in PPG signals respectively corresponding to the more than two types of light beams is calculated, and then oxygen saturation SpO2 is obtained through conversion.

When the wearable electronic device is used to continuously measure the SpO2 for a long time or periodically measure the SpO2, the light source needs to be continuously on or periodically on. To ensure relatively good quality of the PPG detection signal, the PD detection current of the photoelectric detector needs to reach a specific amplitude, and further, the drive current of the light source needs to reach a corresponding amplitude. Therefore, in a scenario in which continuous or periodic measurement is performed, power consumption of an existing PPG detection module is relatively high. This significantly reduces a battery life of the wearable electronic device. In addition, blood oxygen detection fails frequently because the electronic device is not worn in a standard manner, and anti-interference performance of the PPG detection module is also relatively poor.

## SUMMARY

This application provides a blood oxygen detection method and apparatus with multiple photoelectric detectors connected in parallel, to resolve a prior-art problem that power consumption for performing continuous measurement or periodic blood oxygen detection is excessively high because a drive current of a PPG detection module is relatively large, and a battery life of a product is reduced. In addition, blood oxygen detection fails due to non-standard wearing, and anti-interference performance is poor.

To achieve the foregoing objective, the following technical solutions are used in this application.

According to a first aspect, a blood oxygen detection method with multiple photoelectric detectors connected in parallel is provided, and is applied to an electronic device. The electronic device is provided with at least one light source and multiple photoelectric detectors PDs connected in parallel. The method includes: determining, based on a plurality of pieces of detection data, a category of a scenario in which the electronic device currently performs blood oxygen detection, where the detection data is used for describing whether detection currents output by corresponding photoelectric detectors are abnormal, or the detection data is used for describing a current wearing state of the electronic device; and determining, based on the scenario category, to perform calculation based on detection currents output by at least one of the multiple PDs to obtain oxygen saturation, and/or prompting, based on the detection data, a user to re-adjust the wearing state.

In the foregoing technical solution, the electronic device can determine, based on detection data collected in real time, the category of the scenario in which blood oxygen detection is currently performed. In addition, the electronic device, by disposing multiple PD detection modules, can determine, from the multiple PD detection modules connected in parallel, based on the scenario category, detection data of at least one PD on which a user performs blood oxygen detection calculation. The PD may be a PD working normally or a PD with a relatively strong received PPG signal. In a scenario in which continuous measurement or periodic measurement is performed, multiple PD detection modules connected in parallel can effectively reduce an excitation current of a light source, thereby reducing power consumption of the electronic device. In addition, calculating a blood oxygen value based on a detection current obtained by multiple PDs connected in parallel can further improve accuracy of blood oxygen detection.

In a possible design manner, the detection data includes at least one of a photoplethysmography PPG signal corresponding to red light, a PPG signal corresponding to infrared light, a PPG signal corresponding to green light, or a PD detection current, where the PD detection current includes an electrical signal obtained through photoelectric conversion on an optical signal that is received by at least one PD and that is generated after a light beam emitted by at least one light source is reflected by human tissue of a detected user.

In the foregoing possible implementation, the electronic device may determine, based on at least one of a red light PPG signal, an infrared light PPG signal and a green light PPG signal which are collected, or a PD detection current, whether a corresponding PD detection module works normally, to determine a PD for calculating a blood oxygen value, and a PD detection current corresponding to the PD, thereby improving accuracy of blood oxygen detection.

In a possible design manner, the detection data includes monitoring data obtained by an inertial sensor, and is used to describe whether a current wearing state of the electronic device meets a wearing specification.

In the foregoing possible implementation, the electronic device may determine, based on the data collected by the inertial sensor, whether a current wearing state of the electronic device is abnormal, so as to determine, based on the wearing state, whether the current PD detection data can be used, and further generate prompt information based on the wearing state, to notify the user of a reason for the detection abnormality, thereby improving efficiency of interaction with the user and improving user experience.

In a possible design manner, the determining, based on a plurality of pieces of detection data, a category of a scenario in which the electronic device currently performs blood oxygen detection specifically includes: determining, based on the plurality of pieces of detection data and preset thresholds corresponding to the detection data, whether PDs corresponding to the detection data are abnormal; and determining, based on whether a quantity or proportion of normal PDs in the multiple PDs meets a preset condition, the category of the scenario in which the electronic device currently performs blood oxygen detection.

In the foregoing possible implementation, the electronic device may determine, based on the several types of detection data in the foregoing example and with reference to the preset threshold, whether the corresponding PD is abnormal, so that the abnormal PD can be detected from multiple dimensions. In addition, the electronic device may determine a scenario category based on the quantity of normal PDs or a proportion of normal PDs, to classify different blood oxygen detection calculation manners or detection exception processing manners in each scenario category, thereby improving flexibility of processing blood oxygen detection by the electronic device.

In a possible design manner, when detection data corresponding to multiple PDs connected in parallel meets a corresponding preset threshold, it is determined that a category of a scenario in which the electronic device currently performs blood oxygen detection is a first category, and the determining, based on the scenario category, to perform calculation based on a detection current output by at least one of the multiple PDs to obtain oxygen saturation specifically includes: determining a parallel-connected PD detection current corresponding to the multiple PDs connected in parallel as an output current to perform oxygen saturation calculation, to obtain a corresponding oxygen saturation parameter.

In the foregoing possible implementation, when determining, based on the detection data, that all PDs are currently normal, the electronic device may calculate a current blood oxygen value based on detection currents output by multiple configured PDs connected in parallel, so that a drive current required by a light source is relatively low, thereby effectively reducing power consumption of blood oxygen detection and improving a battery life of the wearable electronic device. In addition, the accuracy of blood oxygen calculation can be improved effectively by calculating the blood oxygen value through detection with multiple PDs.

In a possible design manner, when the quantity of normal PDs or a proportion of normal PDs in the multiple PDs is less than a first threshold, it is determined that a category of a scenario in which the electronic device currently performs blood oxygen detection is a second category, and the determining, based on the scenario category, to perform calculation based on a detection current output by at least one of the multiple PDs to obtain oxygen saturation, and/or prompting, based on the detection data, a user to re-adjust the wearing state specifically includes: prompting, based on a layout location of an abnormal PD in the multiple PDs, the user with a reason for abnormity detection, and/or reminding the user to re-adjust the wearing state.

In the foregoing possible implementations, when most PDs of the electronic device detect an exception, a cause of the detected exception and a corresponding user reminder can be obtained based on a layout location of the abnormal PD, so that the user can accurately adjust a wearing manner, thereby improving user experience.

In a possible design manner, the category of the scenario in which the electronic device currently performs blood oxygen detection is determined as a third category when the quantity or proportion of normal PDs in the multiple PDs is greater than or equal to a first threshold, and the determining, based on the scenario category, to perform calculation based on a detection current output by at least one of the multiple PDs to obtain oxygen saturation, and/or prompting, based on the detection data, a user to re-adjust the wearing state specifically includes: determining a PD detection current corresponding to a normal PD in the multiple PDs connected in parallel as an output current to perform oxygen saturation calculation, to obtain a corresponding oxygen saturation parameter; and prompting, based on a layout location of an abnormal PD in the multiple PDs, the user with a reason for abnormity detection, and/or reminding the user to re-adjust a wearing state.

In the foregoing possible implementations, when a PD is detected to be abnormal, the electronic device may further calculate a blood oxygen value based on a detection current of a normal PD, thereby improving anti-interference and flexibility of blood oxygen detection. In addition, a detected exception cause and a corresponding user reminder can be further obtained based on a layout location of the abnormal PD, so that the user can accurately adjust a wearing manner, thereby improving user experience.

In a possible design, the reason for the abnormity detection includes that the electronic device is in at least one of the following states: partial oppression, wearing rollover, side tilting, or overall upward arching.

In the foregoing possible implementation, the electronic device may determine, based on a layout location of the PD exception, a specific exception that may be currently detected, such as partial oppression, wearing rollover, side tilting, or overall upward arching, to accurately instruct the user to adjust a wearing manner, improve blood oxygen detection efficiency, and improve user experience.

In a possible design, spacings between at least two of the multiple PDs and the light source are different.

In the foregoing possible implementation, the electronic device may arrange multiple PDs with different spacings between the PD and the light source, so as to meet detection scenarios of different detection depths of the user, and improve anti-interference performance and flexibility of blood oxygen detection of the electronic device.

In a possible design manner, before the determining, based on the scenario category, to perform calculation based on a detection current output by at least one of the multiple PDs to obtain oxygen saturation, the method further includes: determining, based on temperature data of a current environment in which blood oxygen detection is performed, that the scenario category is a short-spacing category or a long-spacing category; and the determining, based on the scenario category, to perform calculation based on a detection current output by at least one of the multiple PDs to obtain oxygen saturation specifically includes: if the scenario category is a short-spacing category, obtaining oxygen saturation through calculation based on a detection current output by at least one PD that is selected from the multiple PDs and that has a relatively short distance from the light source; or if the scenario category is a long spacing category, obtaining oxygen saturation through calculation based on a detection current output by at least one PD that is selected from the multiple PDs and that has a relatively long distance from the light source.

In the foregoing possible implementation, the electronic device may determine, based on the collected temperature data of the current environment, scenarios of different detection depths in which the user performs blood oxygen detection, so as to select a PD with a relatively long detection depth or a PD with a relatively short detection depth to perform the blood oxygen detection or calculate a final blood oxygen value. This feature effectively improves the accuracy of SpO2 detection in specific scenarios and improves the flexibility of SpO2 detection.

According to a second aspect, a blood oxygen detection apparatus with multiple photoelectric detectors connected in parallel is provided, where at least one light source and multiple photoelectric detectors PDs connected in parallel are configured in the apparatus, and the apparatus includes: a detection module, configured to determine, based on a plurality of pieces of detection data, a category of a scenario in which the apparatus currently performs blood oxygen detection, where the detection data is used to describe whether a detection current output by a corresponding photoelectric detector is abnormal, or the detection data is used to describe a current wearing state of the apparatus; and a processing module, configured to: determine, based on the scenario category, to perform calculation based on a detection current output by at least one of the multiple PDs to obtain oxygen saturation, and/or prompt, based on the detection data, the user to re-adjust a wearing state.

In a possible design manner, the detection data includes at least one of a photoplethysmography PPG signal corresponding to red light, a PPG signal corresponding to infrared light, a PPG signal corresponding to green light, or a PD detection current, where the PD detection current includes an electrical signal obtained through photoelectric conversion on an optical signal that is received by at least one PD and that is generated after a light beam emitted by at least one light source is reflected by human tissue of a detected user.

In a possible design manner, the detection data includes monitoring data obtained by an inertial sensor, and is used to describe whether a current wearing state of the apparatus meets a wearing specification.

In a possible design manner, the detection module is specifically configured to determine, based on the plurality of pieces of detection data and preset thresholds corresponding to the detection data, whether the PD corresponding to the detection data is abnormal; and determine, based on whether the quantity of normal PDs in the multiple PDs or a proportion of normal PDs meets a preset condition, a category of a scenario in which the apparatus currently performs blood oxygen detection.

In a possible design manner, when detection data corresponding to multiple PDs connected in parallel all meets corresponding preset thresholds, it is determined that the category of the scenario in which the apparatus currently performs blood oxygen detection is a first category, and the processing module is specifically configured to: determine a parallel-connected PD detection current corresponding to multiple PDs connected in parallel as output currents, and perform oxygen saturation calculation to obtain a corresponding oxygen saturation parameter.

In a possible design manner, when the quantity of normal PDs in the multiple PDs or the proportion of normal PDs is less than the first threshold, it is determined that the category of the scenario in which the apparatus currently performs blood oxygen detection is the second category, and the processing module is specifically configured to: based on the layout location of the abnormal PD in the multiple PDs, remind the user of a cause of abnormality detection, and/or remind the user to re-adjust the wearing state.

In a possible design manner, when the quantity of normal PDs or the proportion of normal PDs in the multiple PDs is greater than or equal to the first threshold, it is determined that the category of the scenario in which the apparatus currently performs blood oxygen detection is the third category, and the processing module is specifically configured to: determine a PD detection current corresponding to a normal PD in multiple PDs connected in parallel as an output current, perform oxygen saturation calculation, and obtain a corresponding oxygen saturation parameter; and prompt, based on a layout location of an abnormal PD in the multiple PDs, a cause of abnormal detection to the user, and/or remind the user to re-adjust a wearing state.

In a possible design manner, a reason for abnormality detection includes that the apparatus is in at least one of local compression, wearing rollover, side tilting, or overall upward arching.

In a possible design, spacings between at least two of the multiple PDs and the light source are different.

In a possible design, the detection module is further configured to determine, based on the temperature data of the current environment in which blood oxygen detection is performed, that the scenario category is a short-spacing category or a long-spacing category; and the processing module is specifically configured to: if the scenario category is the short-spacing category, obtain, through calculation, oxygen saturation based on a detection current output by at least one PD that is selected from the multiple PDs and that has a relatively short distance from the light source; or if the scenario category is a long spacing category, obtain the oxygen saturation through calculation based on a detection current output by at least one PD that is selected from the multiple PDs and that has a relatively long distance from the light source.

According to a third aspect, an electronic device is provided. The electronic device includes: a processor; and a memory configured to store an executable instruction of the processor, where the processor is configured to execute the instruction, to implement the method according to any implementation of the first aspect.

According to a fourth aspect, a computer readable storage medium is provided. When an instruction in the computer readable storage medium is executed by a processor of an electronic device, the electronic device is enabled to perform the method according to any implementation of the first aspect.

According to a fifth aspect, a computer program product is provided. When the computer program product runs on a computer, the computer is enabled to perform the method according to any implementation of the first aspect.

It may be understood that any one of the blood oxygen detection apparatus with multiple photoelectric detectors connected in parallel, the electronic device, the computer readable storage medium, and the computer program product provided above may be implemented by using the corresponding method provided above. Therefore, for beneficial effects that can be achieved by the apparatus, the electronic device, the computer readable storage medium, and the computer program product, refer to beneficial effects in the corresponding method provided above. Details are not described herein again.

**BRIEF DESCRIPTION OF DRAWINGS**

FIG. 1 is an architecture diagram of a system of an electronic device according to an embodiment of this application;
FIG. 2 is a schematic flowchart of a blood oxygen detection method with multiple photoelectric detectors connected in parallel according to an embodiment of this application;
FIG. 3 is a schematic diagram of a layout of multiple photoelectric detectors connected in parallel according to an embodiment of this application;
FIG. 4 is a schematic diagram of a blood oxygen detection method with multiple photoelectric detectors connected in parallel according to an embodiment of this application;
FIG. 5 is a schematic diagram of another blood oxygen detection method with multiple photoelectric detectors connected in parallel according to an embodiment of this application;
FIG. 6 is a schematic flowchart of a possible blood oxygen detection method with multiple PDs according to an embodiment of this application;

FIG. 7 is a schematic diagram of a possible PD detection current of blood oxygen detection with multiple PDs according to an embodiment of this application;

FIG. 8 is a schematic flowchart of another possible blood oxygen detection method with multiple PDs according to an embodiment of this application; and

FIG. 9 is a schematic diagram of a blood oxygen detection apparatus with multiple photoelectric detectors connected in parallel according to an embodiment of this application.

## DESCRIPTION OF EMBODIMENTS

The terms "first" and "second" mentioned below are merely intended for a purpose of description, and shall not be understood as an indication or implication of relative importance or implicit indication of the number of indicated technical features. Therefore, a feature limited by "first" or "second" may explicitly indicate or implicitly include one or more such features. In the descriptions of embodiments, unless otherwise specified, "multiple" means two or more.

It should be noted that, in this application, terms such as "an example" or "for example" are used for representing giving an example, an illustration, or a description. Any embodiment or design scheme described as "an example" or "for example" in this application should not be explained as being more preferred or having more advantages than another embodiment or design scheme. Exactly, usage of the word "an example", "for example", or the like is intended to present a related concept in a specific manner.

The following clearly and completely describes the technical solutions in embodiments of this application with reference to the drawings in embodiments of this application. It is clear that the described embodiments are merely some but not all of embodiments of this application. All other embodiments obtained by a person of ordinary skill in the art based on embodiments of this application without creative efforts shall fall within the protection scope of this application.

First, an implementation environment and an application scenario of embodiments of this application are briefly described.

A blood oxygen detection method with multiple photoelectric detectors provided in embodiments of this application may be applied to an electronic device having a blood oxygen detection function, where the electronic device may be specifically a wearable device, for example, a smartwatch, an electronic wristband, smart glasses, or smart sneakers.

The wearable device is a portable electronic device that can be worn by a user, or integrated into clothes or accessories of the user. The wearable device may record and display data such as a movement path, a health index, or a living habit of the user, and the wearable device not only is a hardware device, but also can implement a relatively strong data processing function through software support, data interaction, and cloud interaction.

Embodiments of this application provide a blood oxygen detection method with multiple photoelectric detectors, which may be applied to an implementation scenario in which the wearable device performs oxygen saturation detection. Specifically, when measuring oxygen saturation by using the wearable device, the user may perform blood oxygen detection according to the solution in which multiple photoelectric detectors are connected in parallel provided in embodiments of this application, thereby effectively reducing a drive current of a PPG detection module and reducing power consumption of the wearable device. In addition, in the embodiments provided in this application, the wearable device may determine, based on parameters such as a detection current exception currently collected in real time or a state of wearing the wearable device by the user, which of the detection data output by the multiple photoelectric detectors for calculation, to obtain a relatively accurate SpO2 measurement result, thereby effectively improving anti-interference performance of the product. Besides, the wearable device may further remind the user to adjust a wearing state to improve user experience.

A specific form of the foregoing electronic device is not specially limited in the embodiments of this disclosure. The following embodiments are described merely by using an example in which the electronic device is a smartwatch.

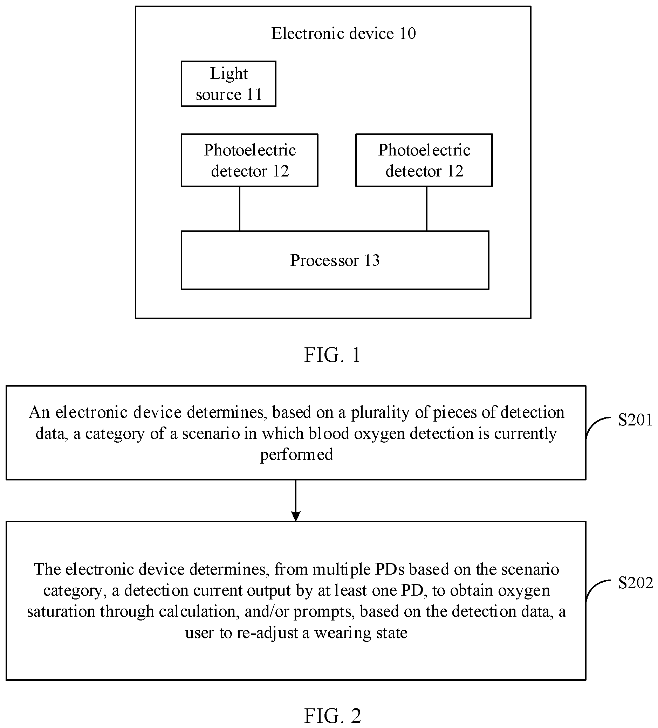

FIG. 1 is an example of a schematic diagram depicting a structure of an electronic device, and the electronic device may be configured to detect oxygen saturation. As shown in FIG. 1, the electronic device 10 includes at least one light source 11, at least two photoelectric detectors 12, and a processor 13.

The light source 11 is used to emit light beams, and the light beams may include red light, infrared light, green light, or other light beams. Absorption coefficients of beams with different wavelengths vary greatly. At present, the red light and the infrared light are usually used for bispectrum quantitative analysis detection. The visible wavelength of the red light is generally 600 nm to 700 nm, and the infrared light refers to invisible light with a wavelength between 760 nm and 1 mm and a frequency lower than that of the red light. For example, the light source in embodiments of this application may emit the red (red) light with a wavelength of 660 nm and the infrared (Infrared, IR) light with a wavelength of 940 nm.

The light source 11 may be specifically a device that can emit light beams of specific wavelengths, such as a laser or a light emitting diode (Light Emitting Diode, LED). For example, the light source 11 may specifically include a red light laser and an infrared light laser, or the light source 11 may include a laser that can emit the red light and the infrared light. A specific form of the light source 11 is not limited in this application.

When the user performs blood oxygen detection by using the electronic device, for example, the light source 11 emits light beams after the user wears the smartwatch on the wrist based on a wearing guide and performs a corresponding operation of starting blood oxygen detection. For example, the light source 11 intermittently emits the red light and the infrared light. The emitted light beams pass through the skin of the user, and are transmitted to human tissue such as adipose tissue, muscle tissue, blood, or bones beneath the skin. At the same time, a portion of reflected optical signals may be generated.

The photoelectric detectors 12 are used to detect the reflected optical signals mentioned above, and perform photoelectric conversion based on the reflected optical signals to obtain specific electrical signals, that is, PD detection currents in this application.

A principle of performing blood oxygen detection in embodiments of this application is that oxygen saturation is obtained by performing a related conversion operation based on the PD detection currents corresponding to light of different wavelengths detected by the photoelectric detectors 12.

Specifically, a ratio of HbO2 to Hb in blood of the detected user may be calculated according to the Beer-Lambert law (Beer Lambert law) with reference to the photoplethysmography PPG mentioned above. The Beer-Lambert law, as the basic law of a spectrophotometric method, describes a relationship between the intensity of absorption of a substance for light of a specific wavelength and the concentration of a light-absorbing substance and the thickness of a liquid layer of the light-absorbing substance.

For example, when the blood oxygen detection is performed, the light source 11 of the electronic device emits the red light and the infrared light; and after the optical signal is reflected by the detected human tissue and received by the photoelectric detectors 12, a DC component and an AC component of a red light PPG signal and a DC component and an AC component of an infrared light PPG signal may be obtained. In this case, a ratio of Hb to HbO2 in the blood of the detected user

$$R = \frac{AC_{red}/DC_{red}}{AC_{IR}/DC_{IR}}$$

meets the following relationship:
, where R represents a parameter of the ratio of Hb to HbO2 in the blood of the detected user, $AC_{red}$ represents the AC component of the red light PPG signal, $DC_{red}$ represents the DC component of the red light PPG signal, $AC_{IR}$ represents the AC component of the infrared light PPG signal, $DC_{IR}$ represents the DC component of the infrared light PPG signal, and the ratio of the AC component to the DC component in the PPG signal may be referred to as a perfusion index (Perfusion Index, PI) value.

Further, after the parameter R of the ratio of Hb to HbO2 in the blood of the detected user is obtained by using an algorithm mentioned above, R can be converted into an oxygen saturation SpO2 parameter according to an empirical mapping function or a specific conversion algorithm. This is not specifically limited in this application. A person skilled in the art may obtain a result by referring to a related algorithm in the conventional technology.

The processor 13 may be configured to: process data obtained by at least one photoelectric detector 12, obtain the oxygen saturation parameter by using a preset algorithm, and control another component of the electronic device to output the oxygen saturation parameter.

For example, the electronic device 10 may further include a display screen, a loudspeaker, and the like. The oxygen saturation parameter obtained by the processor 13 through calculation may be displayed on the display screen, so that the user obtains a detection result by using a visual interface. Alternatively, the loudspeaker may be used to output the oxygen saturation parameter obtained by the processor 13 through calculation, so that the user obtains the detection result through voice output.

It may be understood that the schematic structure in the embodiment of this application does not constitute a specific limitation on an electronic device having a blood oxygen detection function. In some other embodiments of this application, the electronic device having a blood oxygen detection function may include more or fewer components than those shown in the figure, or combine some components, or split some components, or have different component arrangements. The components shown in the figure may be implemented by using hardware, software, or a combination of software and hardware.

It should be noted that the blood oxygen detection performed by the user by using the electronic device may include single blood oxygen detection, continuous blood oxygen detection, or periodic blood oxygen detection.

The single blood oxygen measurement refers to blood oxygen measurement that needs to be manually triggered by the user. If the user manually triggers once, blood oxygen detection module configured in the electronic device works to measure the oxygen saturation once. The continuous blood oxygen measurement means that the electronic device continuously measures the oxygen saturation within a specified time period through setting. For example, the specified time period is from 23:00 to 06:00. In this specified time period, the blood oxygen detection module configured in the electronic device continuously works, and the oxygen saturation is continuously output. The periodic blood oxygen measurement means that the electronic device measures blood oxygen once at intervals of a period of time based on a preset period. For example, the electronic device may be configured to perform blood oxygen measurement once at intervals of 1 hour.

On the basis of the hardware mentioned above in embodiments of this application, the following describes embodiments of this application in detail with reference to the drawings. As shown in FIG. 2, embodiments of this application are applied to an electronic device configured with at least one light source and multiple photoelectric detectors PDs connected in parallel. Embodiments of this application provides a blood oxygen detection method with multiple photoelectric detectors. The method may include the following steps.

S201: The electronic device determines, based on a plurality of pieces of detection data, a category of a scenario in which blood oxygen detection is currently performed.

The detection data is used to describe whether a photoelectric detector corresponding to the detection data is abnormal. For example, the detection data may include a red light PPG signal, an infrared light PPG signal, or a PD detection current, or the like obtained by the photoelectric detector. Whether the detection data is abnormal can be determined by determining whether a size of the detection data meets a preset range. Thus, whether the working state of the photoelectric detector corresponding to the detection data is abnormal may be determined.

It may be learned from a blood oxygen algorithm mentioned above that the red light PPG signal, the infrared light PPG signal, the PD detection current, or the like directly affects calculation of an oxygen saturation parameter. Therefore, whether the detection data is abnormal directly affects the accuracy of outputting a blood oxygen value, or directly affects whether the current blood oxygen detection can successfully output a detection result.

In a possible implementation, the detection data may be also used to describe a wearing state of the electronic device. For example, the detection data may include detection data obtained by an inertial sensor configured in the electronic device. The inertial sensor is a sensor that can be configured to detect and measure acceleration, tilt, shock, vibration, rotation, and a multi-degree-of-freedom motion state of the electronic device. The inertial sensor may include measurement units such as an acceleration sensor and/or an angular velocity sensor (also referred to as a gyroscope).

Accordingly, the electronic device can determine, based on the detection data obtained by the inertial sensor, a wearing state of a user with the current wearable electronic device, and whether the wearable electronic device is not correctly worn according to the wearing specification. For example, whether a smartwatch is worn in a rollover state and arched upward as a whole, or the smartwatch is in a state of continuously flipping, or moving, or the like currently. The wearing state of the electronic device may affect accuracy of outputting a blood oxygen value, or directly affect whether a detection result can be successfully output during current blood oxygen detection.

Whether the detection data is normal or in an abnormal state may affect accuracy of blood oxygen detection performed by the electronic device and whether a value can be successfully obtained through blood oxygen detection. Therefore, the electronic device may determine, based on the detection data mentioned above, a category of a scenario in which blood oxygen detection is currently performed, so as to determine, based on different scenario categories, different blood oxygen detection manners or calculation parameters of the blood oxygen detection.

It should be noted that the electronic device may determine, based on multiple types of detection data mentioned above, scenario categories in which blood oxygen detection is currently performed. The scenario categories may be classified into two categories: normal detection and abnormal detection. For example, when all the detection data is normal, the current scenario is determined to be normal detection; and when one or more of the foregoing detection data is abnormal, the current scenario is determined to be abnormal detection. Alternatively, the scenario classification may be more refined, with more than two scenario categories. For example, the scenario categories may be classified into a first category, a second category, a third category, or the like based on the quantity or proportion of anomalies in multiple detection data. This is not specifically limited in this application. Several classification manners of scenario categories and corresponding blood oxygen detection calculation manners are described as examples in subsequent embodiments.

S202: The electronic device determines, from multiple PDs based on the scenario category, a detection current output by at least one PD, to obtain oxygen saturation through calculation, and/or prompts, based on the detection data, the user to re-adjust a wearing state.

In embodiments of this application, the electronic device is configured with at least one light source and at least two photoelectric detectors PDs connected in parallel. In other words, when a user uses the electronic device to start blood oxygen detection, the light source intermittently emits light beams. In a normal detection mode, the at least two PDs connected in parallel detect reflected light beams. Therefore, the at least two PDs perform photoelectric conversion based on detected reflected optical signals, to obtain PD detection currents. Compared with the conventional technology, when amplitudes of the obtained PD detection currents are the same and the losses of light beams passing through human tissue are the same, a drive current required by the light source in the electronic device is smaller than that required by a PD in the conventional technology, thereby reducing power consumption of blood oxygen detection. The possible reasons are as follows.

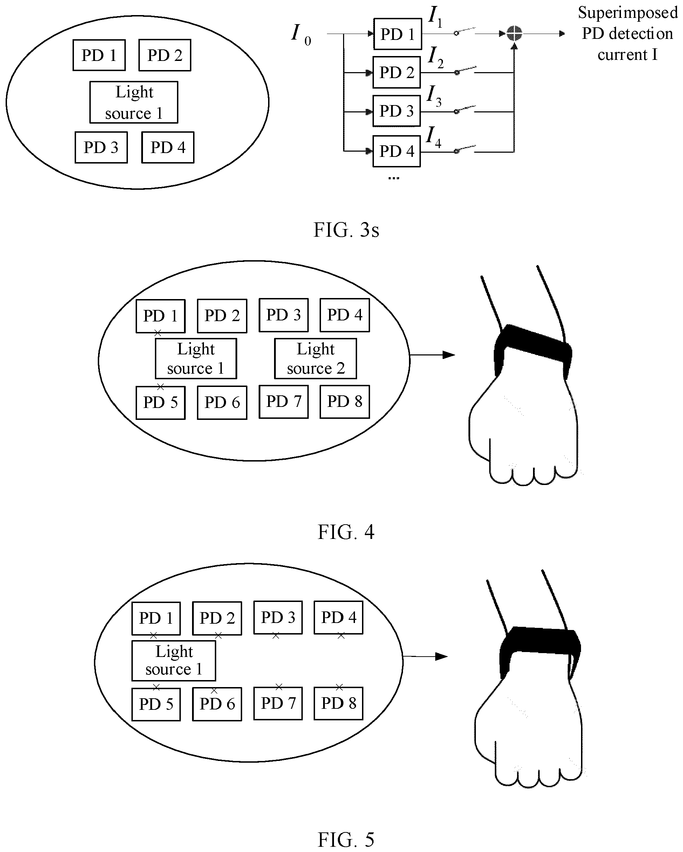

As shown in FIG. 3, the electronic device is configured with a light source 1 and a PD 1 to a PD 4. It is assumed herein that spacings between the PD 1 to the PD 4 and the light source 1 are the same. A PD detection current I required for performing blood oxygen detection needs to reach a specific amplitude, for example, 1 mA. Correspondingly, it is assumed that the PD detection current obtained by performing photoelectric conversion on the optical signal reflected by the light beam emitted by the light source through the human tissue can reach an amplitude of 1 mA. In this case, the drive current of the light source needs to reach a corresponding amplitude, for example, 4 mA. In the foregoing implementation of this application, as shown in FIG. 3, the PD detection current I accumulated by the four PDs connected in parallel needs to reach, for example, an amplitude of 1 mA. In this case, a PD detection current $I_1$-$I_4$ of a single PD in the four PDs connected in parallel may be 0.25 mA. It can be learned from the foregoing assumption that the drive current of the light source needs to reach more than 1 mA to enable the PD detection current obtained by performing photoelectric conversion on the optical signal reflected by the light beam emitted by the light source through the human tissue to reach an amplitude of 0.25 mA. Accordingly, the layout of PDs connected in parallel can effectively reduce the drive current of the light source, thereby reducing power consumption of a wearable electronic device for blood oxygen detection, and improving user experience.

In addition, in the foregoing implementations of this application, signals detected by the at least two PDs may be accumulated, so as to calculate oxygen saturation and also improve calculation accuracy.

In the foregoing embodiment of this application, in the abnormity detection mode, a PD detection current of one PD may be weak and the PD may be considered as an abnormal PD. In this case, the electronic device may determine, from multiple PDs, a PD detection current output by at least one PD, to obtain oxygen saturation through calculation. For example, the detection current of the abnormal PD is not used, or detection data of the abnormal PD is replaced according to an empirical algorithm, so as to output a relatively accurate blood oxygen value through calculation.

Specifically, the detection data of the abnormal PD may be replaced based on the detection data corresponding to the PD that is adjacently disposed or symmetrically disposed with the abnormal PD, to perform corresponding oxygen saturation calculation, thereby improving calculation accuracy.

In addition, the electronic device may locate a current abnormal position of the electronic device based on a layout position of a PD corresponding to the abnormal detection data. Alternatively, the electronic device may detect that the wearing state of the user does not meet a requirement, and the electronic device may notify the user of an abnormal current wearing state by using a screen display manner or a voice prompt manner, and may guide the user to correctly wear the electronic device based on an abnormal status of the detected data. For example, the user is prompted to wear the electronic device again, or the user is guided to adjust the wearing manner based on a layout location of the abnormal PD. Several specific cases are described below as examples, and details are not described herein again.

In the foregoing implementations of this application, the electronic device can determine, based on detection data collected in real time, a category of a scenario in which blood oxygen detection is currently performed. In addition, by disposing multiple PD detection modules, the electronic device can determine, based on the scenario category from the multiple PD detection modules connected in parallel, detection data of at least one PD on which the user performs blood oxygen detection calculation. Multiple PD detection modules connected in parallel can effectively reduce an excitation current of the light source, thereby reducing power consumption of the electronic device. In addition, the accuracy of blood oxygen detection can be further improved.

In an implementation, the type of the detection data in step S201 may specifically include at least one of a red light PPG signal, an infrared light PPG signal, a green light PPG signal, and a PD detection current obtained by a photoelectric detector, or monitoring data obtained by an inertial sensor.

The red light PPG signal represents a reflected signal corresponding to the red light emitted by the light source and detected by the photoelectric detector; the infrared light PPG signal represents a reflected signal corresponding to the infrared light emitted by the light source and detected by the photoelectric detector; and the green light PPG signal represents a reflected signal corresponding to green light emitted by the light source and detected by the photoelectric detector. The electronic device may be further configured with a light source that can emit a green light signal, and the emitted green light is used to detect the PD module or to detect the strength of a current detection signal.

The PD detection current may be an electrical signal output by one PD or multiple PDs connected in parallel, and is used for further calculation of oxygen saturation. Specifically, the processor may further obtain the oxygen saturation parameter through calculation based on PD detection currents output by multiple PDs and PD detection currents output by multiple PDs connected in parallel in a superposition manner.

Whether the red light PPG signal or the infrared light PPG signal is normal may be determined by determining whether a feature of the PPG signal meets a preset threshold, for example, whether an amplitude, a crest interval, a ratio of an upper amplitude to a lower amplitude, a perfusion index PI value, or the like of the PPG signal meets a preset threshold. Whether the green light PPG signal is normal may be determined by determining an amplitude and a crest interval of the green light PPG signal, a correlation between the green light PPG signal and red light/infrared light, and the like.

For example, in an implementation, the electronic device can determine that the PD is normal only when determining that the foregoing four types of detection data are all normal; otherwise, the electronic device determines that the PD is abnormal.

In a specific implementation, in the foregoing step S201, when the electronic device determines that a value of detection data of any of the foregoing several detection data types corresponding to a PD exceeds a corresponding preset threshold, the electronic device determines that the PD is abnormal.

For example, if a detection current of a PD in multiple PDs connected in parallel of the electronic device is less than a specific threshold, the PD detection current may not be used to calculate an output current that is finally superposed by the PDs connected in parallel, and a calculation result of oxygen saturation is not affected.

In another implementation, if a red light PPG signal corresponding to a PD in multiple parallel-connected PDs of the electronic device is less than a specific threshold, a PD detection current corresponding to the PD may be calculated based on a PD detection current at a symmetric position or an adjacent position of the PD.

In an implementation, the determining, based on a plurality of pieces of detection data, a category of a scenario in which blood oxygen detection is currently performed in step S201 may specifically include the following several cases.

1. When the detection data corresponding to all the foregoing PDs is normal, in this case, the electronic device determines that all PDs connected in parallel that currently perform blood oxygen detection work normally, and the scenario category is a normal scenario, that is, the first category.

Correspondingly, in step S202, that the electronic device determines, based on the scenario category from the multiple PDs connected in parallel, the detection current output by the at least one PD for calculation specifically includes: the electronic device may superpose and output the parallel-connected PD detection currents, to calculate oxygen saturation. In this case, an excitation current required by a light source in the electronic device is relatively low, and power consumption is relatively low.

2. When the quantity of normal PDs or the proportion of normal PDs in the detection data corresponding to the multiple PDs is less than the first threshold, the electronic device determines that the category of the scenario in which blood oxygen detection is currently performed is an abnormal scenario, that is, the second category.

The quantity of normal PDs is the quantity of remaining PDs in all PDs configured for the electronic device except the abnormal PD determined based on the detection data in step S201. The proportion of normal PDs is the proportion of the quantity of normal PDs to the quantity of all PDs configured for the electronic device. For example, a total of eight PDs are configured for the electronic device, and it is determined, based on the detection data, that six PDs currently work abnormally. In this case, the proportion of normal PDs is 2/8=25%.

For example, when the first threshold is configured as that the quantity of normal PDs is 2, it indicates that when the quantity of normal PDs is less than 2, the electronic device determines that the category of the scenario in which blood oxygen detection is currently performed is the second category. Alternatively, when the first threshold is configured as that the proportion of normal PDs is 30%, it indicates that when the proportion of normal PDs is less than 30%, the electronic device determines that the category of the scenario in which blood oxygen detection is currently performed is the second category.

Correspondingly, step S202 specifically includes: the electronic device may extract a related feature based on the PD exception, determine a problem scenario to which the PD exception belongs, and notify the user with a reason for abnormity detection and an adjustment manner.

3. When the quantity or proportion of normal data in the plurality of pieces of detection data is greater than or equal to the first threshold, but not all the pieces of detection data are normal, the electronic device determines that the category of the scenario in which blood oxygen detection is currently performed is an abnormal scenario, that is, the third category.

Correspondingly, in step S202, that the electronic device determines, based on the scenario category from the multiple PDs connected in parallel, the detection current output by the at least one PD for calculation specifically includes: the electronic device may calculate oxygen saturation by using the PD detection current output by the at least one normal PD; and the electronic device may determine a reason for abnormity detection by using the abnormal PD, and notify the user.

In a specific implementation, a specific implementation in which the electronic device notifies the user based on the abnormal PD includes: the electronic device can determine a reason for current abnormality detection based on a layout location of the abnormal PD and with reference to a correspondence between a PD layout location in the electronic device and an appearance location of the electronic device, and notify, by using a display screen of the electronic device or a voice prompt, the user of a reason for a measurement failure and how to adjust a wearing manner or a measurement posture, thereby improving user experience.

There is a correspondence between a PD layout position in the electronic device and an appearance position of the electronic device. For example, as shown in FIG. 4, the location of a PD 1 corresponds to the corner of the electronic device, locations of the PD 1, a PD 2, a PD 3, and a PD 4 correspond to one side of the electronic device, and locations of a PD 5, a PD 6, a PD 7, and a PD 8 correspond to the other side of the electronic device.

In an implementation, abnormal detection states include but are not limited to partial oppression, wearing rollover, overall upward arching, or the like. For example, the following several abnormity detection reasons and corresponding prompt information may be specifically included.

In a first case, the electronic device determines the current wearing state as a state of side tilting or rollover based on the position of the abnormal PD.

If one or more PDs on a side of the multiple PDs connected in parallel are abnormal, the electronic device may determine that the current wearing state of the electronic device may be side tilting or rollover. For example, as shown in FIG. 4, if the PD 1 and the PD 5 are abnormal, the electronic device determines that the device is currently flipped and does not fit the skin of the user, and cannot perform normal detection. In this case, the electronic device may remind the user to adjust the wearing state. Alternatively, it is possible that the PD 1, the PD 2, the PD 5, and the PD 6 are all abnormal, and the electronic device determines that one side of the device is currently tilted.

Specifically, the prompt information may include "the blood oxygen measurement fails due to the current wearing rollover; please wear the device again in a standard manner for detection". In addition, if the current electronic device continuously measures blood oxygen for a long time, non-standard wearing at a moment may result in that a blood oxygen value cannot be updated in real time, and only an output result of a previous measurement can be maintained. In this case, the prompt information may include "the blood oxygen measurement maintains a previous measurement result due to the current wearing with a rollover state, and the problem may be resolved after a user tries to wear the electronic device properly".

In a second case, the electronic device determines the current wearing state as overall upward arching by using the abnormal PD.

If one or more PDs on a side of the multiple PDs connected in parallel are abnormal, the electronic device may determine that the current wearing state of the electronic device may be overall upward arching. For example, as shown in FIG. 5, if PD 1, PD 2, PD 3, PD 4, PD 5, PD 6, PD 7 and PD 8 are all abnormal, the electronic device determines that the current device is overall upward arching and does not fit the skin of the user, and cannot perform normal detection. In this case, the electronic device may remind the user to adjust the wearing state.

Correspondingly, the prompt information may include "the measurement of blood oxygen failed due to the wearing in an overall upward arching state; please properly wear the electronic device again for detection". In addition, if the current electronic device continuously measures blood oxygen for a long time, non-standard wearing at a moment may result in that a blood oxygen value cannot be updated in real time, and only an output result of a previous measurement can be maintained. In this case, the prompt information may include "the current wearing side flip causes that the blood oxygen measurement maintains the previous measurement result, and the problem may be resolved after standard wearing".

In a third case, the electronic device determines the current detection state as partial oppression by using the abnormal PD.

If one or more PDs on a side of the multiple PDs connected in parallel are abnormal, the electronic device may determine that a current wearing state of the electronic device may be a partial oppression state. For example, a position corresponding to the PD on the electronic device is oppressed and cannot be measured, or skin of a position corresponding to the user wearing the electronic device is oppressed and cannot be measured successfully. For example, if the PD 1 in FIG. 4 is abnormal, the electronic device determines that the upper left corner of the current device is partial oppression and cannot be detected normally, and the electronic device may remind the user to adjust the wearing state.

Correspondingly, the prompt information may include "the blood oxygen measurement fails due to partial oppression of the current wearing; please wear the device again in a standard manner for detection". In addition, if the current electronic device continuously measures blood oxygen for a long time, non-standard wearing at a moment may result in that a blood oxygen value cannot be updated in real time, and only an output result of a previous measurement can be maintained. In this case, the prompt information may include "blood oxygen measurement maintains a previous measurement result due to the current wearing in a state of partial oppression, and the problem may be resolved after a user wears the device again in a standard manner".

In an implementation, in step S202, when determining that a detection current corresponding to a PD is abnormal, the electronic device may set the detection current of the PD to 0, and the detection current is not included in the parallel-connected PD detection current for output, that is, the abnormal PD is not used to calculate a final blood oxygen value. Alternatively, the detection current of the PD at the symmetrical position of the abnormal PD may be replaced, and the parallel-connected PD detection current is output. Therefore, the abnormal PD has relatively low impact on a final result of blood oxygen detection, and the final blood oxygen detection result is more accurate.

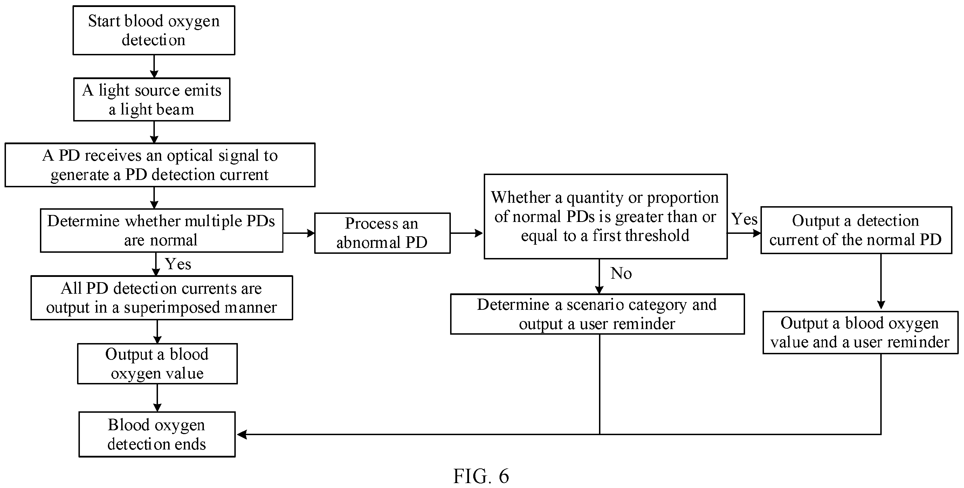

In conclusion, a possible implementation may be shown in FIG. 6. After the electronic device enables the blood oxygen detection function, the light source emits light beams, and all PDs configured in the electronic device receive optical signals to generate a PD detection current. In this case, the electronic device determines, based on detection data corresponding to the multiple PDs, whether the multiple PDs are normal. If all PDs are normal, the detection currents of all PDs are accumulated and output to calculate the blood oxygen value. The blood oxygen value is output, and the blood oxygen detection ends. If the electronic device detects that at least one PD works abnormally, the abnormal PD is processed. The electronic device needs to determine the quantity of normal PDs, and determine a currently detected scenario category by determining whether the quantity or proportion of normal PDs meets a condition. If the quantity or proportion of normal PDs is less than the first threshold, a scenario category of the problematic scenario is determined, and a user reminder is output. If the quantity or proportion of normal PDs is greater than or equal to the first threshold, a detection current accumulated by the normal PDs is output, to calculate a blood oxygen value, determine the scenario category of the problematic scenario, and output the blood oxygen value and the user reminder. The blood oxygen detection ends.

In the foregoing implementation, when the multiple PDs superpose and output the detection current to calculate the blood oxygen value, the drive current required by the light source of the electronic device is reduced, thereby effectively reducing power consumption of blood oxygen detection and improving a battery life of the wearable electronic device. When a specific PD is detected to be abnormal, the electronic device may calculate a blood oxygen value based on a detection current of a normal PD, thereby improving anti-interference performance. In addition, the electronic device can further obtain a reason for the detection exception and a corresponding user reminder based on a layout location of the abnormal PD, so that the user can accurately adjust a wearing manner, thereby improving user experience.

In an implementation, spacings between the multiple PDs configured for the electronic device and the at least one light source may be the same or may be different. By arranging multiple PDs with different spacings from the light source, the electronic device may select a PD combination with an appropriate path length for output, to meet blood oxygen measurement requirements of people with different ambient temperatures or different skin depths. As shown in FIG. 5, pairwise symmetrical PDs may be arranged. A PD 1 and a PD 5 are symmetrical and have the same distance L1 from a light source 1; a PD 2 and a PD 6 are symmetrical, and have the same distance L2 from the light source 1; a PD 3 and a PD 7 are symmetrical, and have the same distance L3 from the light source 1; a PD 4 and a PD 8 are symmetrical, and have the same distance L4 from the light source 1; and L1 < L2 < L3 < L4.

It should be noted that a detection depth of the PPG module is proportional to a distance between the light source and the PD. To be specific, a longer distance between the light source and the PD indicates a larger penetration depth, and a deeper human tissue that light beams emitted by the light source passes through. Therefore, an optical signal received by the PD is generally stronger. When the electronic device performs blood oxygen measurement in an environment with a relatively low temperature, because a capillary of the measured user shrinks, a blood flow decreases or disappears, an AC signal detected by the PPG module is greatly weakened, and accuracy of blood oxygen calculation cannot be ensured. As a result, no value or an incorrect value may be caused. In addition, when blood oxygen measurement is performed for a user with relatively thick skin, due to a limitation of a detection depth of the PPG module, a PD detection signal may be too weak, and blood oxygen detection fails or is inaccurate.

In this embodiment of this application, layout spacings between the multiple PDs and the red/infrared light source may be different. For example, a spacing between the light source and a part of PDs may be set to be within 5 mm. In addition, a spacing between the light source and a part of PDs may be set to be greater than 5 mm, so as to meet a detection requirement of a length and an end spacing.

In an implementation, the electronic device may further obtain the temperature parameter by using the ambient signal collected by the auxiliary module, and by determining whether the temperature parameter meets the second threshold, the electronic device selects, from the multiple PDs, the PD on which blood oxygen detection is currently performed.

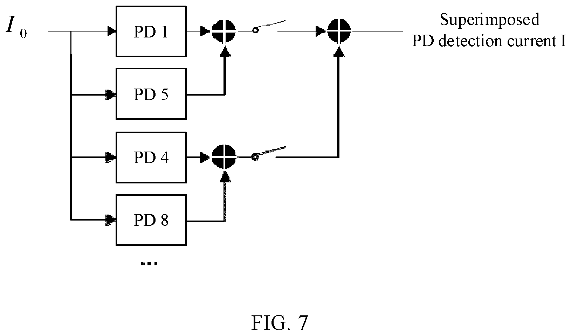

The electronic device may determine the scenario category based on the temperature parameter. If the scenario category is a short-spacing category, the oxygen saturation is obtained through calculation based on a detection current output by at least one PD that is selected from the multiple PDs and that has a relatively short distance from the light source. If the scenario category is a long-spacing category, the oxygen saturation is obtained through calculation based on a detection current output by at least one PD that is selected from the multiple PDs and that has a relatively long distance from the light source. For example, with reference to FIG. 5 and FIG. 7, a superposed PD detection current of the PD 1 and the PD 5 may be output by using the control circuit in the short-spacing category, to obtain oxygen saturation through calculation; and a superposed PD detection current of the PD 4 and the PD 8 may be output by using the control circuit in the long spacing category, to obtain oxygen saturation through calculation.

Specifically, the electronic device may directly collect and calculate the temperature data of the current environment from the temperature sensor, or the electronic device may obtain the temperature data of the current environment by querying a built-in temperature comparison table by using altitude position information collected by a global positioning system (Global Positioning System, GPS) and a barometer. Alternatively, the electronic device may obtain the temperature data of the current environment by using Internet information.

For example, the electronic device may be connected to a smart phone by using a communications module such as Bluetooth, ultrasound, or near field communication (Near Field Communication, NFC). After a blood oxygen detection function is enabled, the electronic device is first connected to the Internet by using the smart phone, then obtains location information by using a GPS of the mobile phone, and further obtains temperature data of a current environment by using the Internet.

In another implementation, the electronic device may further select, from the multiple PDs by using the red light or infrared PPG signal collected by the PD module and by calculating whether a PI value corresponding to the red light or infrared PPG signal meets a third threshold, the PD on which blood oxygen detection is currently performed. PI = AC/DC.

Further, the electronic device may determine the obtained temperature data, to determine whether current blood oxygen detection uses PD detection with a long detection distance or PD detection with a short detection distance, that is, determining that the PD used for blood oxygen detection is enabled.

For example, if the electronic device determines that the current temperature data is greater than a threshold (for example, 10°C), the electronic device may choose to collect red light and infrared PPG signals by using a PD with a relatively short detection distance. A distance from a light source of the PPG module to the PD detector is relatively small, for example, less than 5 mm. Correspondingly, the electronic device converts, based on the PD detection current output by the PD with the short detection distance, the R value into the final oxygen saturation parameter by using a corresponding mapping formula. Alternatively, the electronic device may choose to calculate the blood oxygen value by superposing a PD detection current output by using a PD with a relatively short detection distance and a PD with a relatively long detection distance.

For example, if the electronic device determines that the current temperature data is less than or equal to a threshold (for example, 10°C), the electronic device may choose to collect red light and infrared PPG data by using a PD with a relatively long detection distance. A distance from a light source of the PPG module to the PD detector is relatively long, for example, about 1 cm. Correspondingly, the electronic device converts the R value into the final oxygen saturation parameter based on the PD detection current output by the PD with the long detection distance and by using a corresponding mapping formula.

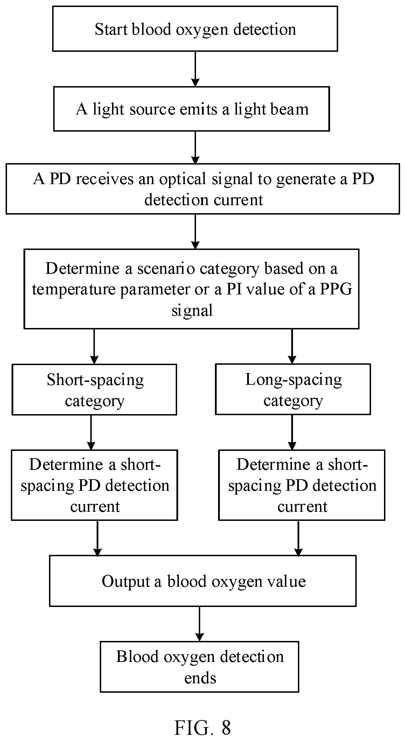

In a possible implementation, as shown in FIG. 8, after the electronic device enables the blood oxygen detection function, the light source emits light beams, and all PDs configured in the electronic device receive the optical signal to generate the PD detection current. In addition, the electronic device obtains a temperature parameter based on a signal collected by the auxiliary module, or obtains a corresponding PI value by using a red light or infrared PPG signal collected by the PD module, and determines, based on the temperature parameter or a current PI value, a category of a scenario in which blood oxygen detection is currently performed. For example, the scenario category is a short-spacing category or a long spacing category, so as to select, based on the scenario category, PD modules to be used and a PD detection current for calculating a blood oxygen value.

According to the foregoing implementations of this application, when the electronic device performs blood oxygen detection, both power consumption and PD detection signal quality can be considered. On the premise of ensuring relatively low power consumption for detection in a normal state, problems of poor PPG signal quality, inaccurate blood oxygen detection, or a low output rate in a low-temperature environment are effectively resolved, thereby improving overall detection accuracy and user experience.

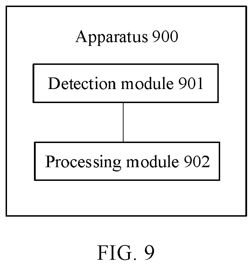

An embodiment of this application further provides a blood oxygen detection apparatus with multiple photoelectric detectors connected in parallel. The apparatus is configured with at least one light source and multiple photoelectric detectors PDs connected in parallel. As shown in FIG. 9, the apparatus 900 may include a detection module 901 and a processing module 902.

The detection module 901 is configured to determine, based on a plurality of pieces of detection data, a category of a scenario in which the apparatus currently performs blood oxygen detection, where the detection data is used to describe whether a detection current output by a corresponding photoelectric detector is abnormal, or the detection data is used to describe a current wearing state of the apparatus.

The processing module 902 is configured to determine, based on the scenario type, to obtain oxygen saturation through calculation based on a detection current output by at least one of the multiple PDs, and/or prompt, based on the detection data, the user to re-adjust a wearing state.

In a possible design manner, the detection data includes at least one of a photovoltaic volume pulse wave notation PPG signal corresponding to red light, a PPG signal corresponding to infrared light, a PPG signal corresponding to green light, or a PD detection current, where the PD detection current includes an electrical signal obtained through photoelectric conversion on an optical signal that is received by at least one PD and that is generated after a light beam emitted by at least one light source is reflected by human tissue of a detected user.

In a possible design manner, the detection data includes monitoring data obtained by an inertial sensor, and is used to describe whether a current wearing state of the apparatus meets a wearing specification.

In a possible design manner, the detection module 901 is specifically configured to: determine, based on preset thresholds corresponding to a plurality of pieces of detection data and the detection data, whether a PD corresponding to the detection data is abnormal; and determine, based on whether the quantity of normal PDs or a proportion of normal PDs in the multiple PDs meets a preset condition, the category of the scenario in which the apparatus currently performs blood oxygen detection.

In a possible design manner, when detection data corresponding to multiple PDs connected in parallel all meets corresponding preset thresholds, it is determined that the category of the scenario in which the apparatus currently performs blood oxygen detection is the first category. In this case, the processing module 902 is specifically configured to determine a parallel-connected PD detection current corresponding to multiple PDs connected in parallel as an output current to perform oxygen saturation calculation, to obtain a corresponding oxygen saturation parameter.

In a possible design manner, when the quantity of normal PDs or a proportion of normal PDs in the multiple PDs is less than a first threshold, it is determined that the category of the scenario in which the apparatus currently performs blood oxygen detection is the second category. The processing module 902 is specifically configured to: prompt, based on a layout location of the abnormal PD in the multiple PDs, the user with a reason for abnormity detection, and/or remind the user to re-adjust the wearing state.

In a possible design manner, when the quantity of normal PDs or a proportion of normal PDs in the multiple PDs is greater than or equal to a first threshold, it is determined that the category of the scenario in which the apparatus currently performs blood oxygen detection is a third category. The processing module 902 is specifically configured to: determine a PD detection current corresponding to a normal PD in the multiple PDs connected in parallel as an output current to perform oxygen saturation calculation, to obtain a corresponding oxygen saturation parameter; and prompt, based on a layout location of the abnormal PD in the multiple PDs, the user with a reason for abnormity detection, and/or remind the user to re-adjust the wearing state.

In a possible design manner, the reason for abnormity detection includes that the apparatus is in at least one of the following states: partial oppression, wearing rollover, side tilting, or overall upward arching.

In a possible design, spacings between at least two of the multiple PDs and the light source are different.

In a possible design manner, the detection module 901 is further configured to determine, based on temperature data of a current environment in which blood oxygen detection is performed, whether the scenario category is a short-spacing category or a long-spacing category.

The processing module 902 is specifically configured to: if the scenario category is a short-spacing category, obtain the oxygen saturation through calculation based on a detection current output by at least one PD that is selected from the multiple PDs and that has a relatively short distance from the light source; or if the scenario category is a long spacing category, obtain the oxygen saturation through calculation based on a detection current output by at least one PD that is selected from the multiple PDs and that has a relatively long distance from the light source.

In addition, for a specific execution process and embodiment of the apparatus 900, refer to the steps performed by the electronic device and related descriptions in the foregoing method embodiments. For a resolved technical problem and a brought technical effect, refer to the content in the foregoing embodiments. Details are not described herein again.

In this embodiment, the apparatus is presented in a form of functional modules obtained through division in an integrated manner. The module" herein may be a specific circuit, a processor and a memory that execute one or more software or firmware programs, an integrated logic circuit, and/or another component that can provide the foregoing functions.

In some embodiments, with reference to FIG. 1, the processor 13 in FIG. 1 may invoke the computer executable instruction stored in the memory, so that a wearable electronic device such as a smartwatch performs the method in the foregoing method embodiment.

For example, functions/implementation processes of the detection module 901 and the processing module 902 in FIG. 9 may be implemented by the processor 13 in FIG. 1 by invoking the computer executable instruction stored in the memory.

In an example embodiment, a storage medium including an instruction is further provided, for example, a memory including an instruction. The instruction may be executed by the processor 13 of the electronic device to complete the foregoing method.

All or some of the foregoing embodiments may be implemented by using software, hardware, firmware, or any combination thereof. When a software program is used to implement the embodiments, all or some of the embodiments may be implemented in a form of a computer program product. The computer program product includes one or more computer instructions. When the computer program instructions are loaded and executed on the foregoing electronic device, the procedures or functions according to embodiments of this application are all or partially generated.

A person skilled in the art can easily figure out another implementation solution of the disclosure after considering the specification and practicing the disclosure that is disclosed herein. This application is intended to cover any variations, functions, or adaptive changes of the disclosure. These variations, functions, or adaptive changes comply with general principles of the disclosure, and include common knowledge or a commonly used technical means in the technical field that is not disclosed in the disclosure. The specification and the embodiments are merely considered as examples, and the actual scope and the spirit of the disclosure are pointed out by the following claims.

In conclusion, the foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

**Claims**

1. A blood oxygen detection method with multiple photoelectric detectors connected in parallel, applied to an electronic device, wherein the electronic device is configured with at least one light source and multiple photoelectric detectors PDs connected in parallel, and the method comprises:

   determining, based on a plurality of pieces of detection data, a category of a scenario in which the electronic device currently performs blood oxygen detection, wherein the detection data is used for describing whether detection currents output by corresponding photoelectric detectors are abnormal, or the detection data is used for describing a current wearing state of the electronic device; and
   determining, based on the scenario category, to perform calculation based on a detection current output by at least one of the multiple PDs to obtain oxygen saturation, and/or prompting, based on the detection data, a user to re-adjust the wearing state.

2. The method according to claim 1, wherein the detection data comprises at least one of a photoplethysmography PPG signal corresponding to red light, a PPG signal corresponding to infrared light, a PPG signal corresponding to green light, or a PD detection current, wherein the PD detection current comprises an electrical signal obtained through photoelectric conversion on an optical signal that is received by at least one PD and that is generated after a light beam emitted by at least one light source is reflected by human tissue of a detected user.

3. The method according to claim 1 or 2, wherein the detection data comprises monitoring data obtained by an inertial sensor, and is used for describing whether the current wearing state of the electronic device meets a wearing specification.

4. The method according to any one of claims 1 to 3, wherein the determining, based on a plurality of pieces of detection data, a category of a scenario in which the electronic device currently performs blood oxygen detection specifically comprises:

   determining, based on the plurality of pieces of detection data and preset thresholds corresponding to the detection data, whether PDs corresponding to the detection data are abnormal; and
   determining, based on whether a quantity or proportion of normal PDs in the multiple PDs meets a preset condition, the category of the scenario in which the electronic device currently performs blood oxygen detection.

5. The method according to claim 4, wherein the category of the scenario in which the electronic device currently performs blood oxygen detection is determined as a first category when detection data corresponding to the multiple PDs connected in parallel all meets corresponding preset thresholds, and the determining, based on the scenario category, to perform calculation based on a detection current output by at least one of the multiple PDs to obtain oxygen saturation specifically comprises:
   determining a parallel-connected PD detection current corresponding to the multiple PDs connected in parallel as an output current to perform oxygen saturation calculation, to obtain a corresponding oxygen saturation parameter.

6. The method according to claim 4, wherein the category of the scenario in which the electronic device currently performs blood oxygen detection is determined as a second category when the quantity or proportion of normal PDs in the multiple PDs is less than a first threshold, and the determining, based on the scenario category, to perform calculation based on a detection current output by at least one of the multiple PDs to obtain oxygen saturation, and/or prompting, based on the detection data, a user to re-adjust the wearing state specifically comprises:
   prompting, based on a layout location of an abnormal PD in the multiple PDs, the user with a reason for abnormity detection, and/or reminding the user to re-adjust the wearing state.

7. The method according to claim 4, wherein the category of the scenario in which the electronic device currently performs blood oxygen detection is determined as a third category when the quantity or proportion of normal PDs in the multiple PDs is greater than or equal to a first threshold, and the determining, based on the scenario category, to perform calculation based on a detection current output by at least one of the multiple PDs to obtain oxygen saturation, and/or prompting, based on the detection data, a user to re-adjust the wearing state specifically comprises:

   determining a PD detection current corresponding to a normal PD in the multiple PDs connected in parallel as an output current to perform oxygen saturation calculation, to obtain a corresponding oxygen saturation parameter; and

prompting, based on a layout location of an abnormal PD in the multiple PDs, the user with a reason for abnormity detection, and/or reminding the user to re-adjust the wearing state.

8. The method according to claim 6 or 7, wherein the reason for abnormity detection comprises that the electronic device is in at least one of the following states: partial oppression, wearing rollover, side tilting, or overall upward arching.

9. The method according to any one of claims 1 to 8, wherein spacings between at least two of the multiple PDs and the light source are different.

10. The method according to claim 9, wherein before the determining, based on the scenario category, to perform calculation based on a detection current output by at least one of the multiple PDs to obtain oxygen saturation, the method further comprises:

> determining, based on temperature data of a current environment in which blood oxygen detection is performed, that the scenario category is a short-spacing category or a long-spacing category; and
> the determining, based on the scenario category, to perform calculation based on a detection current output by at least one of the multiple PDs to obtain oxygen saturation specifically comprises:
>
> > if the scenario category is the short-spacing category, obtaining the oxygen saturation through calculation based on a detection current output by at least one PD that is selected from the multiple PDs and that has a relatively short distance from the light source; or
> > if the scenario category is the long-spacing category, obtaining the oxygen saturation through calculation based on a detection current output by at least one PD that is selected from the multiple PDs and that has a relatively long distance from the light source.

11. A blood oxygen detection apparatus with multiple photoelectric detectors connected in parallel, wherein the apparatus is configured with at least one light source and multiple photoelectric detectors PDs connected in parallel, and the apparatus comprises:

> a detection module, configured to determine, based on a plurality of pieces of detection data, a category of a scenario in which the apparatus currently performs blood oxygen detection, wherein the detection data is used for describing whether detection currents output by corresponding photoelectric detectors are abnormal, or the detection data is used for describing a current wearing state of the apparatus; and
a processing module, configured to determine, based on the scenario category, to perform calculation based on a detection current output by at least one of the multiple PDs to obtain oxygen saturation, and/or prompt, based on the detection data, a user to re-adjust a wearing state.

12. The apparatus according to claim 11, wherein the detection data comprises at least one of a photoplethysmography PPG signal corresponding to red light, a PPG signal corresponding to infrared light, a PPG signal corresponding to green light, or a PD detection current, wherein the PD detection current comprises an electrical signal obtained through photoelectric conversion on an optical signal that is received by at least one PD and that is generated after a light beam emitted by the at least one light source is reflected by human tissue of a detected user.

13. The apparatus according to claim 11 or 12, wherein the detection data comprises monitoring data obtained by an inertial sensor, and is used to describe whether a current wearing state of the apparatus meets a wearing specification.

14. The apparatus according to any one of claims 11 to 13, wherein the detection module is specifically configured to:

> determine, based on the plurality of pieces of detection data and preset thresholds corresponding to the detection data, whether PDs corresponding to the detection data are abnormal; and
> determine, based on whether a quantity or proportion of normal PDs in the multiple PDs meets a preset condition, the category of the scenario in which the apparatus currently performs blood oxygen detection.

15. The apparatus according to claim 14, wherein the category of the scenario in which the apparatus currently performs blood oxygen detection is determined as a first category when detection data corresponding to the multiple PDs connected in parallel all meets corresponding preset thresholds, and the processing module is specifically configured to:
determine a parallel-connected PD detection current corresponding to the multiple PDs connected in parallel as an output current to perform oxygen saturation calculation, to obtain a corresponding oxygen saturation parameter.

16. The apparatus according to claim 14, wherein the category of the scenario in which the apparatus currently performs blood oxygen detection is determined as a second category when the quantity or proportion of normal PDs in the multiple PDs is less than a first threshold, and the processing module is specifically configured to:
prompt, based on a layout location of an abnormal PD in the multiple PDs, the user with a reason for abnormity detection, and/or remind the user to re-adjust the wearing state.

17. The apparatus according to claim 14, wherein the category of the scenario in which the apparatus currently performs blood oxygen detection is determined as a third category when the quantity or proportion of normal PDs in the multiple PDs is greater than or equal to a first threshold, and the processing module is specifically configured to:

    determine a PD detection current corresponding to a normal PD in the multiple PDs connected in parallel as an output current to perform oxygen saturation calculation, to obtain a corresponding oxygen saturation parameter; and
    prompt, based on a layout location of an abnormal PD in the multiple PDs, the user with a reason for abnormity detection, and/or remind the user to re-adjust the wearing state.

18. The apparatus according to claim 16 or 17, wherein the reason for abnormity detection comprises that the apparatus is in at least one of the following states: partial oppression, wearing rollover, side tilting, or overall upward arching.

19. The apparatus according to any one of claims 11 to 18, wherein spacings between at least two of the multiple PDs and the light source are different.

20. The apparatus according to claim 19, wherein the detection module is further configured to:

    determine, based on temperature data of a current environment in which blood oxygen detection is performed, that the scenario category is a short-spacing category or a long-spacing category; and
    the processing module is specifically configured to:

       if the scenario category is the short-spacing category, obtain the oxygen saturation through calculation based on a detection current output by at least one PD that is selected from the multiple PDs and that has a relatively short distance from the light source; or
       if the scenario category is the long-spacing category, obtain the oxygen saturation through calculation based on a detection current output by at least one PD that is selected from the multiple PDs and that has a relatively long distance from the light source.

21. An electronic device, wherein the electronic device comprises:

    a processor, and
    a memory, configured to store an executable instruction of the processor, wherein
    the processor is configured to execute the instruction, to implement the method according to any one of claims 1 to 10.

22. A computer readable storage medium, wherein when an instruction in the computer readable storage medium is executed by a processor of an electronic device, the electronic device is enabled to perform the method according to any one of claims 1 to 10.

23. A computer program product, wherein a computer is enabled to perform the method according to any one of claims 1 to 10 when the computer program product runs on the computer.

Electronic device 10

Light source 11

Photoelectric detector 12

Photoelectric detector 12

Processor 13

FIG. 1

An electronic device determines, based on a plurality of pieces of detection data, a category of a scenario in which blood oxygen detection is currently performed — S201

The electronic device determines, from multiple PDs based on the scenario category, a detection current output by at least one PD, to obtain oxygen saturation through calculation, and/or prompts, based on the detection data, a user to re-adjust a wearing state — S202

FIG. 2

$I_0$    PD 1 — $I_1$    Superimposed
PD detection
PD 2 — $I_2$    current I
PD 3 — $I_3$
PD 4 — $I_4$
...

FIG. 3s

FIG. 4

FIG. 5

```
┌─────────────────────┐
│  Start blood oxygen │
│      detection      │
└─────────────────────┘
           │
           ▼
┌─────────────────────┐
│  A light source emits│
│     a light beam    │
└─────────────────────┘
           │
           ▼
┌──────────────────────────┐
│ A PD receives an optical │
│ signal to generate a PD  │
│    detection current     │
└──────────────────────────┘
           │
           ▼
┌──────────────────────────┐      ┌──────────────┐      ┌──────────────────────────┐      ┌──────────────────────┐
│ Determine whether multiple│────▶│ Process an   │────▶│ Whether a quantity or    │ Yes  │ Output a detection   │
│    PDs are normal         │      │ abnormal PD  │      │ proportion of normal PDs │─────▶│ current of the normal │
└──────────────────────────┘      └──────────────┘      │ is greater than or equal │      │         PD           │
           │ Yes                                         │   to a first threshold   │      └──────────────────────┘
           ▼                                             └──────────────────────────┘                  │
┌──────────────────────────┐                                        │ No                              ▼
│ All PD detection currents │                                        ▼                     ┌──────────────────────┐
│ are output in a           │                             ┌──────────────────────────┐    │ Output a blood oxygen│
│ superimposed manner       │                             │ Determine a scenario     │    │ value and a user     │
└──────────────────────────┘                             │ category and output a    │    │ reminder             │
           │                                             │ user reminder            │    └──────────────────────┘
           ▼                                             └──────────────────────────┘                  │
┌─────────────────────┐                                              │                                │
│  Output a blood     │                                              │                                │
│   oxygen value      │                                              │                                │
└─────────────────────┘                                              │                                │
           │                                                         │                                │
           ▼                                                         │                                │
┌─────────────────────┐                                              │                                │
│  Blood oxygen       │◀─────────────────────────────────────────────┴────────────────────────────────┘
│  detection ends     │
└─────────────────────┘
```

FIG. 6

$I_0$ PD 1

PD 5

PD 4

PD 8

...

Superimposed
PD detection current I

FIG. 7

Start blood oxygen detection

↓

A light source emits a light beam

↓

A PD receives an optical signal to generate a PD detection current

↓

Determine a scenario category based on a temperature parameter or a PI value of a PPG signal

↓

Short-spacing category

Long-spacing category

↓

Determine a short-spacing PD detection current

Determine a short-spacing PD detection current

↓

Output a blood oxygen value

↓

Blood oxygen detection ends

FIG. 8

Apparatus 900

Detection module 901

Processing module 902

FIG. 9

## INTERNATIONAL SEARCH REPORT

| International application No. |
| --- |
| **PCT/CN2021/110415** |

**A. CLASSIFICATION OF SUBJECT MATTER**

A61B 5/1455(2006.01)i

According to International Patent Classification (IPC) or to both national classification and IPC

**B. FIELDS SEARCHED**

Minimum documentation searched (classification system followed by classification symbols)

A61B5

Documentation searched other than minimum documentation to the extent that such documents are included in the fields searched

Electronic data base consulted during the international search (name of data base and, where practicable, search terms used)

CNTXT, WPABSC, ENTXTC: AC交流, DC直流, 电流, 探测, PPG, 光电, 检测, PD, 并联, 血氧, 光电容积脉搏, 传感器; VEN, WPABS, USTXT, WOTXT, EPTXT: AC, DC, CURRENT, DETECT+, PHOTOPLETHYSMOGRAPHY, PHOTOELECTRIC, VOLUME, PULSE, PPG, PHOTODETECTOR?, PARALLEL, OXYGEN, SATURATION, SPO2, SENSOR

**C. DOCUMENTS CONSIDERED TO BE RELEVANT**

| Category* | Citation of document, with indication, where appropriate, of the relevant passages | Relevant to claim No. |
| --- | --- | --- |
| PX | CN 112741626 A (HUAWEI TECHNOLOGIES CO., LTD.) 04 May 2021 (2021-05-04) claims 1-22, description paragraphs 58-152, figures 1-9 | 1-23 |
| Y | CN 111134648 A (HUAWEI DEVICE CO., LTD.) 12 May 2020 (2020-05-12) description paragraphs 5, 53-59, 85-104, 121-123 and figures 1-12 | 1-23 |
| Y | US 2017014035 A1 (SANMINA CORP.) 19 January 2017 (2017-01-19) description, paragraphs 65-70, 92-101 and figures 1-6 | 1-23 |
| Y | WO 2019019029 A1 (HUAWEI TECHNOLOGIES CO., LTD.) 31 January 2019 (2019-01-31) description page 6 line 35 to page 8 line 9, page 11 line 9 to page 12 line 8 and figures 1-18 | 1-23 |
| Y | US 2017189629 A1 (SANMINA CORP.) 06 July 2017 (2017-07-06) description, paragraphs 78-128 and figures 10-12 | 1-23 |
| Y | US 2017181678 A1 (SANMINA CORP.) 29 June 2017 (2017-06-29) description paragraphs 93, 116-151 and figures 17-19 | 1-23 |

☑ Further documents are listed in the continuation of Box C.    ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **18 October 2021** | **04 November 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**EP 4 205 650 A1**

<table>
<tr><td colspan="2">INTERNATIONAL SEARCH REPORT</td><td>International application No.<br>**PCT/CN2021/110415**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| Y | US 2017215811 A1 (SANMINA CORP.) 03 August 2017 (2017-08-03)<br>description, paragraphs 75-76, 113-148 and figures 17-19 | 1-23 |
| A | WO 2019213874 A1 (COROS SPORTS TECH SHENZHEN CO., LTD.) 14 November 2019 (2019-11-14)<br>entire document | 1-23 |
| A | US 2020187788 A1 (IMEC VZW et al.) 18 June 2020 (2020-06-18)<br>entire document | 1-23 |
| A | CN 109924960 A (DO TECHNOLOGY CO., LTD.) 25 June 2019 (2019-06-25)<br>entire document | 1-23 |

Form PCT/ISA/210 (second sheet) (January 2015)

| International application No. |
|---|
| **PCT/CN2021/110415** |

| Patent document cited in search report | | | Publication date (day/month/year) | Patent family member(s) | | | Publication date (day/month/year) |
|---|---|---|---|---|---|---|---|
| CN | 112741626 | A | 04 May 2021 | None | | | |
| CN | 111134648 | A | 12 May 2020 | EP | 3851029 | A1 | 21 July 2021 |
| | | | | WO | 2020088639 | A1 | 07 May 2020 |
| | | | | CN | 111134648 | B | 04 May 2021 |
| US | 2017014035 | A1 | 19 January 2017 | US | 9642538 | B2 | 09 May 2017 |
| | | | | US | 2017215751 | A1 | 03 August 2017 |
| | | | | US | 9974451 | B2 | 22 May 2018 |
| | | | | CA | 2999410 | A1 | 30 March 2017 |
| | | | | CA | 2999410 | C | 27 August 2019 |
| | | | | EP | 3337397 | A1 | 27 June 2018 |
| | | | | EP | 3399913 | A2 | 14 November 2018 |
| | | | | WO | 2017120615 | A2 | 13 July 2017 |
| | | | | US | 2017014572 | A1 | 19 January 2017 |
| | | | | WO | 2017054006 | A1 | 30 March 2017 |
| | | | | US | 9636457 | B2 | 02 May 2017 |
| | | | | EP | 3337390 | A1 | 27 June 2018 |
| | | | | WO | 2017053925 | A1 | 30 March 2017 |
| | | | | US | 9642578 | B2 | 09 May 2017 |
| | | | | EP | 3337390 | B9 | 07 July 2021 |
| | | | | WO | 2017053926 | A1 | 30 March 2017 |
| | | | | CN | 108024727 | A | 11 May 2018 |
| | | | | EP | 3834710 | A1 | 16 June 2021 |
| | | | | US | 2017071550 | A1 | 16 March 2017 |
| | | | | CN | 108024745 | A | 11 May 2018 |
| | | | | EP | 3337394 | A1 | 27 June 2018 |
| | | | | IN | 201817009972 | A | 06 July 2018 |
| | | | | US | 10744262 | B2 | 18 August 2020 |
| | | | | US | 2019076601 | A1 | 14 March 2019 |
| | | | | US | 9980676 | B2 | 29 May 2018 |
| | | | | US | 2017215793 | A1 | 03 August 2017 |
| | | | | US | 2017274146 | A1 | 28 September 2017 |
| | | | | US | 10155087 | B2 | 18 December 2018 |
| | | | | EP | 3337390 | B1 | 06 January 2021 |
| | | | | WO | 2018057058 | A1 | 29 March 2018 |
| WO | 2019019029 | A1 | 31 January 2019 | US | 2020146629 | A1 | 14 May 2020 |
| | | | | CN | 110785118 | A | 11 February 2020 |
| | | | | EP | 3632301 | A1 | 08 April 2020 |
| US | 2017189629 | A1 | 06 July 2017 | US | 10500354 | B2 | 10 December 2019 |
| US | 2017181678 | A1 | 29 June 2017 | US | 10750981 | B2 | 25 August 2020 |
| US | 2017215811 | A1 | 03 August 2017 | US | 2018055454 | A1 | 01 March 2018 |
| | | | | US | 10231674 | B2 | 19 March 2019 |
| | | | | US | 10932727 | B2 | 02 March 2021 |
| | | | | US | 2021137466 | A1 | 13 May 2021 |
| WO | 2019213874 | A1 | 14 November 2019 | None | | | |
| US | 2020187788 | A1 | 18 June 2020 | EP | 3666180 | A1 | 17 June 2020 |
| | | | | EP | 3666180 | B1 | 14 July 2021 |
| CN | 109924960 | A | 25 June 2019 | None | | | |

Form PCT/ISA/210 (patent family annex) (January 2015)

**EP 4 205 650 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010943525